# EUROPÄISCHE PATENTSCHRIFT

(11) **EP 1 972 287 B1**
(45) Veröffentlichungstag und Bekanntmachung des Hinweises auf die Patenterteilung: **12.09.2012**
(21) Anmeldenummer: 08001475.6
(22) Anmeldetag: 26.01.2008
(51) Int. Cl.: A61B 17/16, A61C 1/14, B23B 31/12

(54) **Spülbares Spannfutter**
Rinsable chuck
Mandrin rinceable

(30) Priorität: 17.03.2007 DE 102007012859
(43) Veröffentlichungstag der Anmeldung: 24.09.2008
(73) Patentinhaber: Josef Albrecht Bohrfutterfabrik GmbH & Co. KG, 73249 Wernau (DE)
(72) Erfinder: Schwarz, Dieter, 73061 Ebersbach (DE); Schmideder, Martin, 70771 Leinfelden-Echterdingen (DE)
(74) Vertreter: Rüger, Barthelt & Abel

(56) Entgegenhaltungen:
- DE-A1- 10 027 784
- DE-A1- 19 726 942
- DE-A1- 19 848 371
- DE-A1-102005 014 096

## Beschreibung

Die Erfindung betrifft ein Bohr- oder Spannfutter für den Einsatz in medizinischen Geräten.

Insbesondere in der Chirurgie aber auch auf anderen medizinischen Gebieten, werden gelegentlich Geräte benötigt, in denen drehbare Werkzeuge, wie Bohrer, Fräser oder dergleichen zu spannen sind. Dazu dienen Spann- oder Bohrfutter, wie sie beispielsweise aus der DE 26 52 831 A1 oder auch der DE 27 35 358 A1 bekannt sind.

Im Einsatz kommen nicht nur das Werkzeug sondern auch das Spannfutter mit Körperflüssigkeiten und Gewebe des Patienten in Berührung. Nach Gebrauch müssen die Spannfutter gereinigt werden. Dazu dienen geeignete Waschmaschinen.

Bei der Reinigung muss darauf geachtet werden, dass das betreffende Werkzeug oder Spannfutter möglichst rückstandsfrei gereinigt wird.

Aus der DE 197 26 942 A1 ist ein Spannfutter mit einer Bypass-Spülung bekannt. Die Werkzeugmaschine weist eine Arbeitsspindel zur Aufnahme von Werkzeughaltern mit sogenannten Hohlkegelschäften auf. Ein in der Werkzeugspindel vorgesehenes zentrales Spannelement ist dazu eingerichtet, einen zentralen Zapfen der Hohlkegelschaft-Werkzeugaufnahme in eine entsprechende konusförmige Vertiefung der Arbeitsspindel zu ziehen. Das Spannelement enthält einen Fluidkanal, der über Radialbohrungen mit einem in der Arbeitsspindel vorgesehenen Spülkanal in Verbindung steht. Der Spülkanal endet an einer stirnseitigen Mündung der Arbeitsspindel. Der Kanal dient der Zuführung von Kühlmittel zum Werkzeughalter. Beim Umspannen von Hohlkegelschaft-Werkzeughaltern muss die rückwärtige Plananlagefläche des Werkzeughalters gespült werden. Dazu kann aus dem Bypasskanal ein entsprechender Fluidstrahl austreten.

Aus ähnlichen Gründen ist an einem Werkzeughalter nach der DE 198 48 371 ein Spülkanal ausgebildet. Das Spülen von Steilkegelschäften ist zudem aus der DE 10 2005 014 096 A1 bekannt.

Aus der DE 100 27 784 A1 ist ein Spaunfutter gemäß dem Oberbegriff des Anspruches 1 bekannt.

Es ist Aufgabe der Erfindung, ein Bohr- oder Spannfutter zu schaffen, das erhöhten Hygieneanforderungen genügt.

Diese Aufgabe wird mit dem Spannfutter nach Anspruch 1 gelöst:

Das erfindungsgemäße Spannfutter weist ein Gehäuse mit einer Durchgangsbohrung bzw. einem Durchgangskanal auf, der vorzugsweise konzentrisch zur Drehachse des Spannfutters angeordnet ist. Der Durchgangskanal kann z.B. dazu genutzt werden, ein gespanntes Werkzeug mit Fluiden wie Luft, Kochsalzlösung oder anderen Behandlungsmitteln zu versorgen. Bei der Reinigung des Spannfutters kann die Durchgangsbohrung zur Zuleitung von Reinigungsfluid genutzt werden.

Das erfindungsgemäße Bohr- oder Spannfutter weist ein Getriebe mit wenigstens einem Getriebeelement auf, das dazu dient, ein Spannelement zum Festspannen und zum Lösen eines Werkzeugs zu bewegen. Das Getriebeelement kann beispielsweise eine hülsenförmige Druckspindel sein, die in Axialrichtung auf das oder die Spannelemente einwirkt. Vorzugsweise enthält es eine Durchgangsbohrung, die konzentrisch zu der Drehachse des Spannfutters angeordnet ist und von der ein Spülkanal abzweigt. Damit führt ein Spülkanal ausgehend von dem in dem Gehäuse und seinen Einbauten festgelegten zentralen Durchgangskanal in Innenräume des Spannfutters hinein, die sonst von Spülfluiden nur schwer erreicht werden. Der zumindest eine Spülkanal ermöglicht somit die gezielte Herbeiführung einer Durchströmung der Innenräume des Spannfutters zur Reinigung derselben und zur Beseitigung von Geweben, Geweberesten, Resten von Körperflüssigkeiten, wie Blut, Lymphe und dergleichen. Vorzugsweise sind mehrere Spülkanäle vorgesehen, von denen zumindest einer in die Innenräume des Spannfutters hineinführt und von denen mindestens einer aus den Innenräumen des Spannfutters herausführt. Vorzugsweise weist der Durchgangskanal einen Sitz zur Aufnahme eines Spüldorns auf. Dieser Sitz kann beispielsweise durch eine Ringschulter gebildet sein, mit der das Spannfutter auf einer Sitzfläche eines Spüldorns aufsitzt. Vorzugsweise ist dieser Sitz zwischen einem Spülkanal, der Spülfluid in die Innenräume des Spannfutters leitet, und einem Spülkanal angeordnet, der das Spülfluid aus den Innenräumen des Spannfutters wieder ausleitet. Die Spülkanäle verbessern die Effizienz von Waschvorgängen.

Die so beschriebene Lage des Spülkanals und des Abflusskanals bezieht sich insbesondere auf die Axialposition des Spannfutters.

Vorzugsweise ist das Gehäuse des Spannfutters mehrteilig aufgebaut. Es kann z.B. aus hülsenartigen Teilen bestehen, die gegeneinander verdrehbar sind, um dadurch das Spannelement zu spannen oder zu lösen. Das Spannfutter ist als Dreibackenfutter ausgebildet. Zur Bewegung der drei Backen kann ein Getriebeelement in Form einer Druckspindel vorgesehen sein. Vorzugsweise ist diese Druckspindel als Schraubhülse ausgebildet mit einem zentralen Durchgangskanal und einem ungefähr radial abzweigenden Spülkanal. In der Druckhülse kann auch der bereits erwähnte Sitz zur Aufnahme des Spüldorns ausgebildet sein. Um die Druckspindel herum ist ein Ringraum ausgebildet, der als innerer Hohlraum des Spannfutters mit Reinigungsflüssigkeit durchspülbar ist. Der Spülkanal mündet unmittelbar in diesen Ringraum. Der Abflusskanal führt an axial entfernter Stelle aus diesem Ringraum heraus. Durch die Durchspülung des Hohlraums der in dem Spannfutter beispielsweise konzentrisch zu dem Durchgangskanal ausgebildet ist, wird erreicht, dass sich in dem Spannfutter keine versteckten Schmutznester ausbilden können. Insbesondere wird vermieden, dass in versteckten Spalten oder Hohlräumen geringe infektiöse Reste verbleiben, die dann beim Einsatz des Spannfutters beispielsweise durch die einwirkenden mechanischen Kräfte aus ihren Spalten heraus gefördert werden und zu dem Patienten gelangen. Auch wird verhindert, dass sich in Hohlräumen Reste von Waschflüssigkeit halten. Der Spätere Austritt von Waschflüssigkeit oder deren Eintrocknungsresten beim Einsatz des Spannfutters wird unterbunden.

Das Spannfutter ist insbesondere darauf eingerichtet, mit einem Spüldorn zusammen zu wirken, der im Wesentlichen etwa senkrecht angeordnet ist und auf den das Spannfutter aufsteckbar ist. Aus dem stirnseitigen offenen Ende des Spüldorns austretendes Spülfluid durchspült das Spannfutter und erreicht sowohl seine versteckten Innenräume als auch seine Außenfläche, so dass es auf diese Weise besonders gründlich gewaschen wird.

Weitere Einzelheiten vorteilhafter Ausführungsformen der Erfindung ergeben sich aus der Zeichnung, der Beschreibung oder Ansprüchen. Die Beschreibung beschränkt sich auf wesentliche Aspekte der Erfindung und sonstiger Gegebenheiten. Die Zeichnung ist ergänzend heranzuziehen.

In der Zeichnung sind Ausführungsbeispiele der Erfindung veranschaulicht. Es zeigen:
- Figur 1: ein Spannfutter erfindungsgemäßer Bauart in teilweise aufgeschnittener Seitenansicht,
- Figur 2: das Spannfutter nach Figur 1 während eines Spülvorgangs,
- Figur 3: eine abgewandelte Ausführungsform des erfindungsgemäßen Spannfutters in teilweise geschnittener Seitenansicht,
- Figur 4: das Spannfutter nach Figur 3 während des Spülvorgangs,
- Figur 5: eine weitere Ausführungsform des Spannfutters in teilweise geschnittener Seitenansicht,
- Figur 6: eine in dem Spannfutter nach Figur 5 vorgesehene Druckspindel in Seitenansicht,
- Figur 7: die Druckspindel nach Figur 6 in Draufsicht,
- Figur 8: das Spannfutter nach Figur 3 während eines abgewandelten Spülvorgangs und
- Figur 9: einen Spüldorn für eine Waschmaschine zur Aufnahme des erfindungsgemäßen Spannfutters.

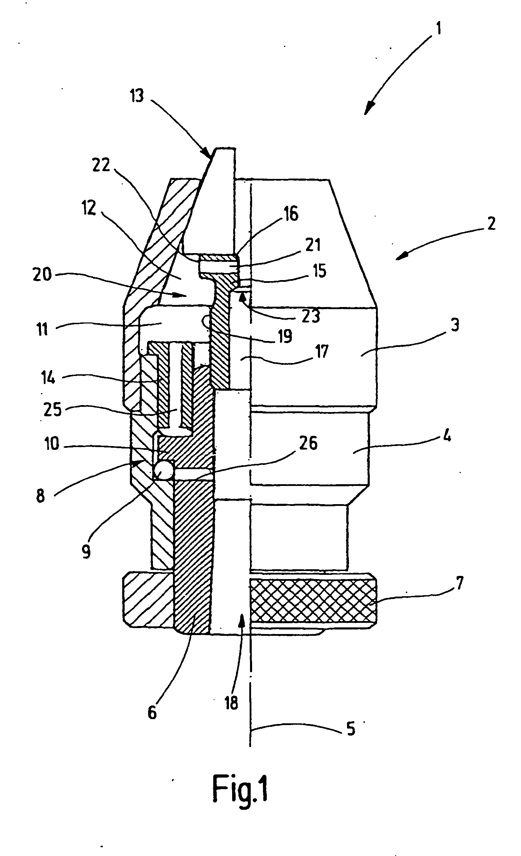

Figur 1 veranschaulicht ein Spannfutter 1 wie es zum Spannen von medizinischen Bohrern, Fräsern oder sonstigen drehend anzutreibenden Werkzeugen an medizinischen Geräten Anwendung finden kann. Das Spannfutter 1 weist ein Gehäuse 2 auf, das in der vorliegenden Ausführungsform mehrteilig aufgebaut ist. Zu ihm gehören z.B. zwei miteinander fest verschraubte Hülsen oder ringförmige Teilgehäuse 3, 4, die konzentrisch zu der Drehachse 5 des Spannfutters 1 angeordnet sind. Das Gehäuse 2 umschließt einen Hohlraum, in dem ein hülsenförmiger Träger 6 angeordnet ist. Dieser trägt an seinem aus dem Gehäuse 2 heraus schauenden Ende einen gerändelten Griffring 7. Dieser ist mit dem Träger 6 drehfest verbunden. Das Teilgehäuse 4 ist auf dem Träger 6 mit geringem Spiel angeordnet und gegen diesen verdrehbar. Zur Lagerung in Radial- und Axialrichtung ist ein Kugellager 8 vorgesehen, dessen Kugeln 9 zwischen einer Ringschulter des Teilgehäuses 4 und einer Ringschulter 10 des Trägers 6 laufen.

In dem Gehäuse 2, das auf dem Träger 3 drehbar ist, ist ein Spannbackenträger 11 angeordnet, der drehfest mit dem Gehäuse 2 verbunden ist. Der Spannbackenträger 11 weist einen vorderen Abschnitt auf, der in einem sich konisch verjüngenden Teil des Teilgehäuses 3 sitzt und Führungen 12 zur Lagerung und Führung von Spannbacken 13 aufweist. In Figur 1 ist lediglich ein einziger Spannbacken 13 veranschaulicht. Es sind jedoch zumindest drei solcher Spannbacken in Abständen von 120° um die Drehachse 5 herum vorgesehen. Entsprechend sind auch zumindest drei solcher Führungen 12 vorgesehen, die durch entsprechende Schlitze in dem Spannbackenträger 11 ausgebildet sind. Alternativ können die Spannbacken 13 auch unmittelbar in dem Teilgehäuse 3 geführt sein, beispielsweise durch entsprechende Nuten an der konischen Innenseite desselben.

Der Spannbackenträger 11 weist einen Sockelabschnitt 14 auf, der mit dem Gehäuse 2 verbunden ist und beispielsweise drehfest in diesem sitzt.

Die Spannbacken 13 sitzen außerdem in entsprechenden Radialschlitzen einer Druckspindel 15, die dazu an ihrer oberen Stirnseite 16 z.B. Radialschlitze aufweisen kann, in denen die Spannbacken 13 sitzen. Die Druckspindel 15 ist vorzugsweise als Hülse ausgebildet. Sie weist eine zentrale Durchgangsbohrung 17 auf, die Teil eines längs durch das Spannfutter 1 führenden und konzentrisch zu der Drehachse 5 angeordneten Durchgangskanals 18 ist. Die Druckspindel 15 ist an ihrem Außenumfang mit einem Gewinde 19 versehen, das mit einem Innengewinde des Trägers 6 in Eingriff steht. Die Druckspindel 15 ist drehfest mit dem Gehäuse 2 verbunden. Die Kopplung kann z.B. über den Spannbackenträger 11 und die Spannbacken 13 erfolgen. Bei einer Verdrehung des Gehäuses 2 gegen den Träger 6 schraubt sich somit die Druckspindel 15 axial in Spann- oder Löserichtung. Entsprechend werden die Spannbacken 13 in Axialrichtung bewegt. Wegen der Konizität des vorderen Gehäuseabschnitts 2 vollführen sie dabei zugleich eine radiale Spannbewegung.

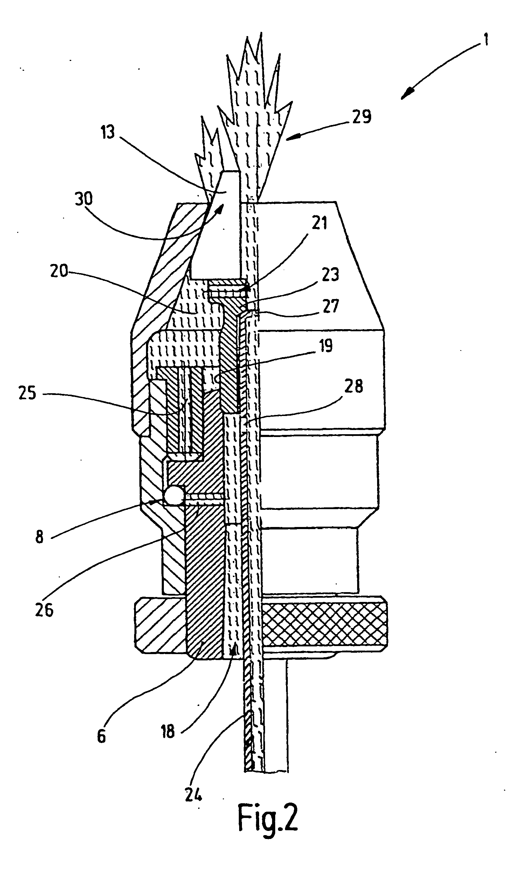

Um den Außenumfang der Druckspindel 15 herum ist in dem Gehäuse 2 ein innerer Hohlraum 20 ausgebildet, in den Verschmutzungen, z.B. in Form von Blut, Lymphflüssigkeit, Gewebeflüssigkeit und Gewebepartikeln gelangen kann, wenn das Spannfutter 1 im chirurgischen Betrieb eingesetzt wird. Zur Reinigung dieses Hohlraums 20 und von mit diesem Hohlraum 20 in Verbindung stehenden Spalten weist die Druckspindel 15 einen Spülkanal 21 auf, der von dem Durchgangskanal 18 abzweigt. Er ist beispielsweise in dem vorderen, die Spannbacken 13 unterstützenden Bund 22 der Druckspindel 15 ausgebildet. Vorzugsweise sind in diesem Bund 22 mehrere entsprechende Spülkanäle z.B. zwei, drei, vier oder noch mehr angeordnet. Die Spülkanäle 21 können Radialbohrungen sein. In unmittelbarer Nachbarschaft zu dem Spülkanal 21, vorzugsweise an der von den Spannbacken 13 abliegenden Seite ist die Durchgangsbohrung 17 der Druckspindel 15 mit einer Stufe 23 versehen, bei der sich der Durchmesser der Durchgangsbohrung 17 verengt. Die Durchgangsbohrung 17 wird somit von dem Griffring 7 ausgesehen zu den Spannbacken 13 hin an der Stufe 23 enger. Die Stufe 23 dient der Anlage eines Spüldorns 24, wie es aus Figur 2 ersichtlich ist.

Das Spannfutter 1 kann weitere Spülkanäle, wie beispielsweise einen Spülkanal 25, der in dem Spannbackenträger 11 parallel zu der Drehachse 5 in Axialrichtung angeordnet ist und einen Spülkanal 26 aufweisen, der z.B. von dem Kugellager 8 abzweigt und durch den Träger 6 in den Durchgangskanal 18 führt. Bedarfsweise können mehrere solcher Spülkanäle 25, 26 vorgesehen sein.

Das insoweit beschriebene Spannfutter 1 kann, wie insbesondere aus Figur 2 hervorgeht, mittels einer automatischen Waschmaschine gründlich gereinigt werden. Dies geschieht wie folgt:

Wie in Figur 2 veranschaulicht, kann eine entsprechende Reinigungs- oder Waschmaschine für das Spannfutter 1 ein Spüldorn 24 aufweisen, der im Wesentlichen vertikal angeordnet ist und dem Spannfutter 1 Spülfluid zuleitet. Das Spülfluid ist in Figur 2 durch eine Wellenschraffur angedeutet. Eine entsprechende Waschmaschine kann eine Vielzahl solcher Spüldorne aufweisen. Der Spüldorn 24 ist an seinem oberen Ende mit einer Spülfluidaustrittsöffnung 27 versehen. Der Durchmesser des Spüldornes 24 ist so bemessen, dass er durch den Durchgangskanal 18 bis zu der Stufe 23 gesteckt werden kann, wobei die Stufe 23 dann auf der Stirnseite des Spüldorns 24 sitzt. Der Spüldorn 27 kann an seinem Außenumfang zusätzlich mit einer oder mehreren Spülfluidaustrittsöffnungen 28 versehen sein. Durch den Spüldorn 24 wird ständig Spülfluid gefördert. Es tritt an seinen Spülfluidaustrittsöffnungen 27, 28 aus. Das stirnseitig an der Spülfluidaustrittsöffnung 27 austretende Spülfluid durchdringt den von den Spannbacken 13 umgebenen Kanal und tritt aus dem Spannfutter 1 stirnseitig aus. Dies ist in Figur 2 durch eine Spülfluidfontäne 29 symbolisiert. Diese umspült die Spannbacken 13 und wäscht die Stirn- und Außenseite des Spannfutters 1 gründlich ab. Zugleich aber dringt das Spülfluid durch den Spülkanal 21 in den Hohlraum 20 und füllt diesen mit Spülfluid. Es dringt dann weiter durch den Spülkanal 25 zu dem Kugellager 8 vor, reinigt dies und fließt dann über den Spülkanal 26 ab. Desgleichen kann Spülfluid aus dem Hohlraum 20 durch den Gewindeeingriff zwischen dem Außengewinde 19 und dem Träger 6 in den Durchgangskanal 18 zurücksickern und damit auch verborgene Spalte wie das Gewinde gründlich reinigen.

Vorzugsweise wird die Reinigung des Spannfutters 1 bei einer vorgegebenen Position der Spannbacken 13 durchgeführt. Dazu kann an dem Spannbacken 13 eine spezielle Marke 30 vorgesehen sein, die der Bediener vor Durchführung des Waschvorgangs in eine bestimmte Sollposition bringen muss. Die Marke 30 kann beispielsweise ein an einem oder allen Spannbacken 13 vorgesehener gravierter Strich oder eine sonstige Markierung sein, die z.B. mit der Stirnfläche des Gehäuses 1 in Fluchtung gebracht werden muss.

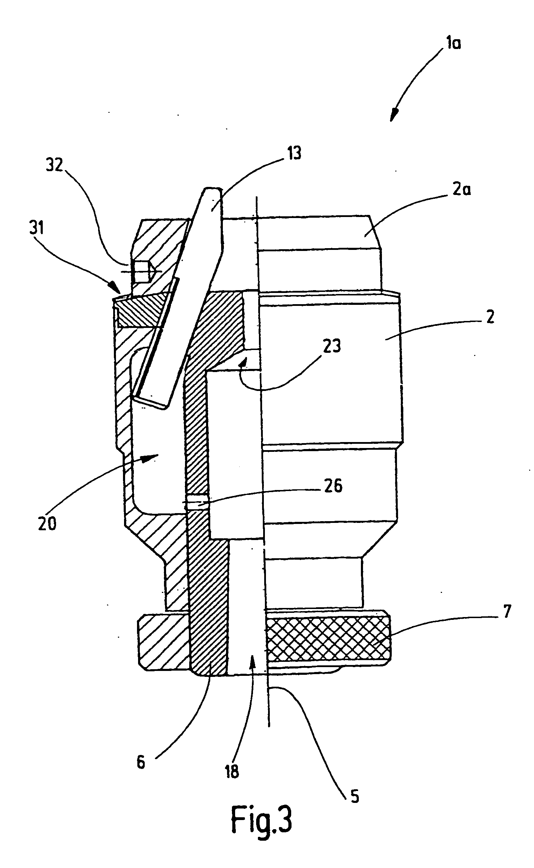

Figur 3 veranschaulicht eine abgewandelte Ausführungsform des Spannfutters 1. Soweit prinzipielle bauliche oder funktionelle Übereinstimmung mit dem vorbeschriebenen Futter 1 besteht, wird unter Zugrundelegung gleicher Bezugszeichen auf die vorige Beschreibung verwiesen. Im Unterschied zu dem vorbeschriebenen Futter, das ein Handspannfutter ist, handelt es sich bei dem Spannfutter 1a um ein Futter für einen Spannschlüssel. Sein Gehäuse 2 sitzt drehbar auf dem Träger 6, an dem die Spannbacken 13 schräg zu der Mittelachse 5 verschiebbar gelagert sind. Der Träger 6 weist an einer frei außen zu Tage liegenden Stelle eine Zahnradverzahnung 31 auf, mit der ein Kegelrad in Eingriff gebracht werden kann. An einem Gehäuseabschnitt 2a in Nachbarschaft der Verzahnung 31 ist eine Bohrung 32 vorgesehen, in die ein Wellenende des Kegelrads eingesteckt werden kann. Dieses steht mit der Verzahnung 31 in Eingriff und gehört zu einem Spannschlüssel. Wird er gedreht verdreht sich der Gehäuseteil 2a gegen den Träger 6, wodurch letztendlich die Spannbacken 13 in der einen oder anderen Axialrichtung bewegt werden, wodurch sie auch eine Radialbewegung ausführen, um ein Werkzeug zu spannen oder freizugeben.

Auch bei dieser Ausführungsform der Erfindung ist wiederum ein zentraler Durchgangskanal 18 vorgesehen, der von dem einen stirnseitigen Ende des Gehäuses 2 zu dem anderen stirnseitigen Ende des Trägers 6 führt. Innerhalb des Durchgangskanals 18 ist wiederum eine Stufe 23 vorgesehen, die als Sitz für den Spüldorn 24 (siehe Figur 2) dient. Der Träger 6 ist mit einem Spülkanal 26 versehen, durch den aus dem Hohlraum 20 austretendes Spülfluid in den Durchgangskanal 18 gelangt.

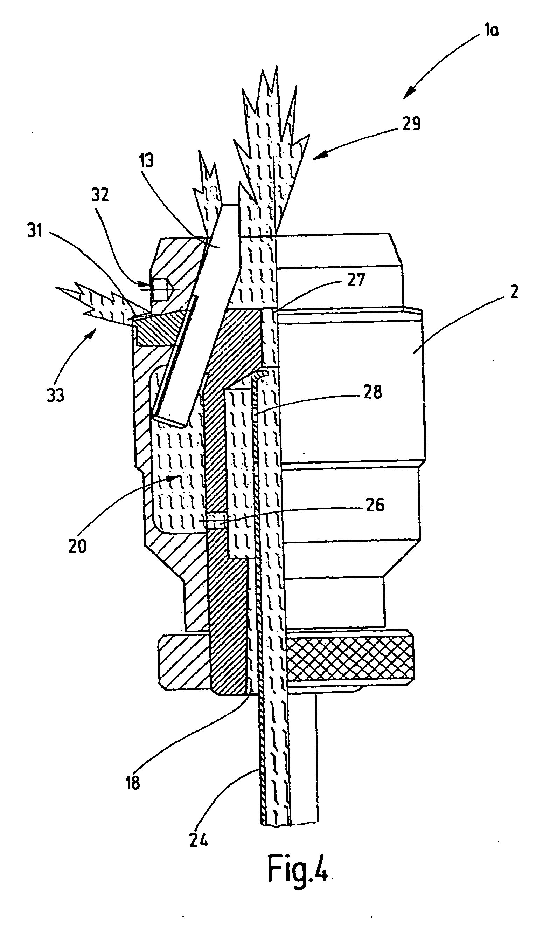

Zur Reinigung des Futters 1a wird, wie Figur 4 zeigt, das Futter auf den Spüldorn 24 aufgesetzt. Das aus der Spülfluidaustrittsöffnung 27 austretende Spülfluid umspült die vorderen aus dem Gehäuse 2 heraus ragenden Enden der Spannbacken 13 und bildet die Spülfluidfontäne 29. Zugleich dringt das Spülfluid über Gehäusespalte in den Hohlraum 20 vor und tritt auch als Spülfluidfontäne 33 an der Verzahnung 31 aus. Das reichlich in den Hohlraum 20 gelangende Spülfluid wäscht die Spalten entlang der Spannbacken 13 sauber und läuft über den Spülfluidkanal 26 in den Durchgangskanal 18 zurück, um dort abzulaufen.

Es wird nach dem Waschvorgang ein auch in seinen tiefen Spalten gereinigtes Spannfutter 1a erhalten.

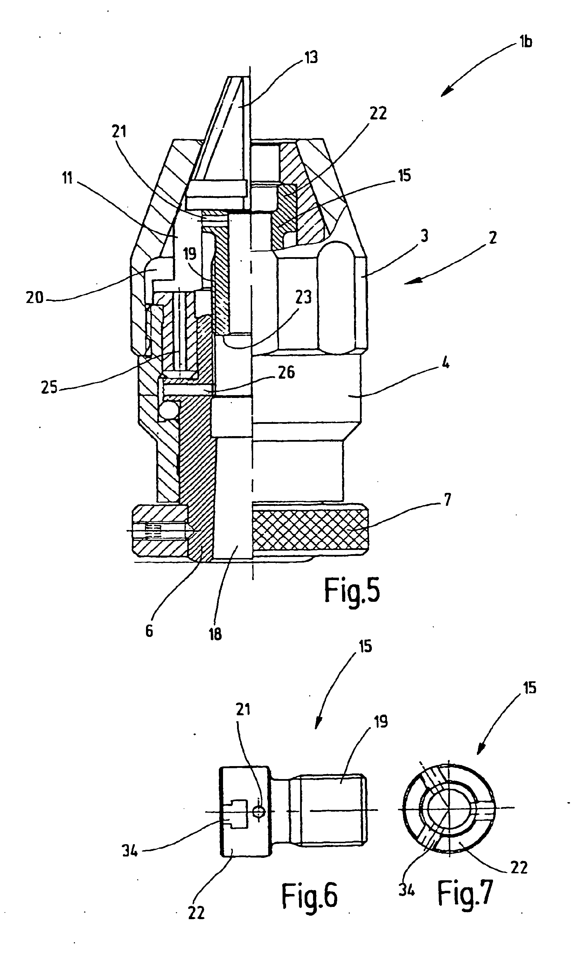

Eine weitere abgewandelte Ausführungsform der Erfindung veranschaulicht Fig. 5 anhand des Spannfutters 1b. Es ist wiederum ein Dreibackenfutter mit Spannbacken 13 und Handbetätigung durch Verdrehung des Gehäuses 2 gegen den Griffring 7. Die Stufe 23 wird hier durch die untere Stirnseite der Druckspindel 15 gebildet, die zur besseren Veranschaulichung in Fig. 6 und 7 nochmals gesondert veranschaulicht ist. Wie ersichtlich, weist sie an ihrem Bund 22 Führungsnuten 34 und zumindest einen Spülkanal 21 auf. Die Funktion ist wie im Zusammenhang mit Fig. 1 und 2 beschrieben.

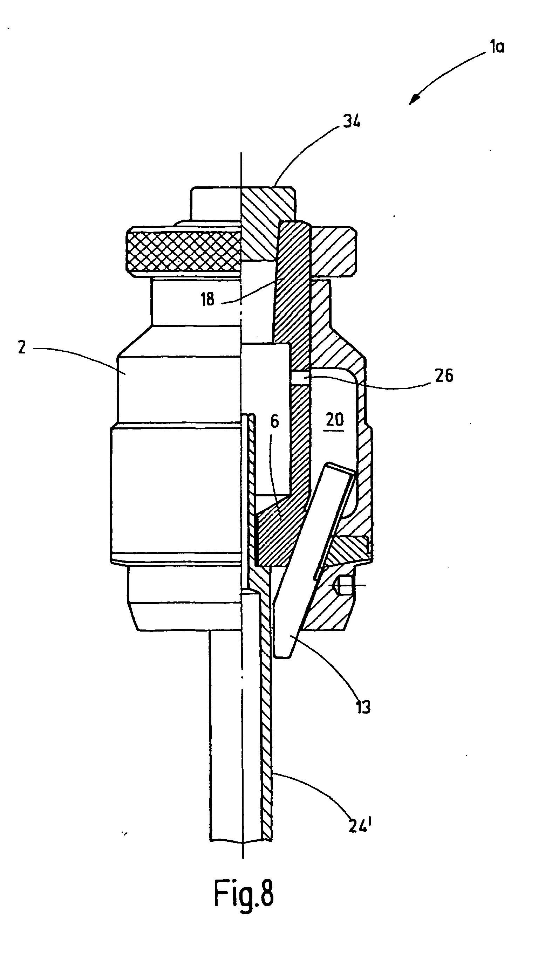

Figur 8 veranschaulicht eine abgewandelte Spülmöglichkeit für das Spannfutter 1a gemäß Figur 3. Das Spannfutter 1a ist mit seiner zur Aufnahme von Werkzeugen eingerichteten Öffnung auf den Spüldorn 24a aufgesetzt. Durch diesen wird sein Innenraum, wie zuvor erläutert, mit Spülfluid beaufschlagt. An seinem Befestigungsende ist das Spannfutter 1a bzw. sein Gehäuse 2 durch ein geeignetes Verschlusselement 34, beispielsweise in Form eines Stopfens verschlossen. Der Spülkanal 26 dient nun zur Einleitung von Spülfluid in den Hohlraum 20, aus dem Spülfluid entlang der Spannbacken 13 wieder ausströmen kann.

Diese Art der Durchspülung des Spannfutters 1a und seines Hohlraumes 20 wird als besonders effizient angesehen.

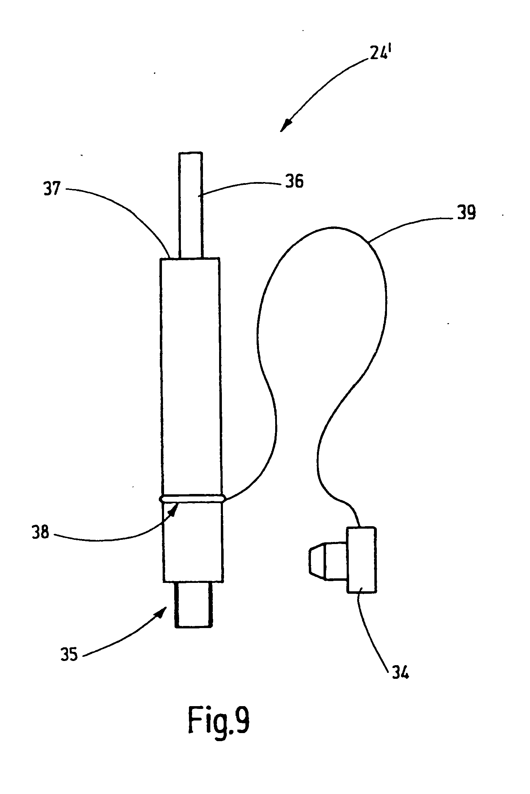

Figur 9 veranschaulicht den Spüldorn 24' gesondert. Er ist durch ein speziell geformtes Rohr gebildet. An seinem in Figur 9 unteren Ende ist der Spüldorn 24' mit einem Verbindungsmittel beispielsweise in Form eines gewindetragenden Abschnitts 35 versehen. Dieser kann in ein Hohlprofil eingeschraubt, mit dem Spülfluid innerhalb der Waschmaschine zu dem Spüldorn 24' herangeführt wird. An Stelle des Abschnitts 35 können auch andere Verbindungsmittel, wie beispielsweise Bajonettverbindungen vorgesehen werden.

An dem anderen Ende des Spüldorns 24' ist ein Rohrfortsatz 36 vorgesehen, dessen Durchmesser geringer ist als der übrige Durchmesser des Spüldorns 24'. Der Rohrfortsatz 36 ist von einer Ringschulter 37 umgeben, die eine Stufe bildet und zugleich eine Anlagefläche festlegt, die der Aufnahme des Spannfutters 1a oder auch des Spannfutters 1 dient. Die Ringschulter 37 bildet einen Sitz für den Träger 6, der einigermaßen abdichtend auf der Ringschulter 37 aufliegt.

Der Spüldorn 24' kann außerdem mit dem Verschlusselement 34 verbunden sein, um dieses unverlierbar zu halten. beispielsweise ist das Verschlusselement 34 über eine Schnur, eine Kette oder ein flexibles Kunststoffstück mit dem Spanndorn 24' verbunden. Beispielsweise kann dieser mit einer Ringnut 38 versehen sein, die mit Kunststoff umspritzt ist, der sich als Faden 39 zu dem Verschlusselement 34 fortsetzt, der ebenfalls aus dem gleichen Kunststoff ausgebildet sein kann. Der die Ringnut 38 füllende Kunststoffring, der Faden 39 und das Verschlusselement 34 können ein einstückiges Kunststoffteil bilden.

Ein für den Einsatz in der Medizin vorgesehenes Spannfutter 1 weist zumindest einen inneren Hohlraum 20 auf, der konzentrisch zu einem längs durchgehenden offenen Kanal 18 vorgesehen sein kann und mit diesem über Klüfte und Spalten kommuniziert. Zumindest ein eigens vorgesehener Spülkanal 21 und/oder 26 gestattet die gezielte Durchspülung dieses inneren Hohlraums beim Waschen des Futters. Es wird eine verbesserte Hygiene erreicht.

### Bezugszeichen

- 1: Spannfutter
- 2: Gehäuse
- 3, 4: Teilgehäuse
- 5: Drehachse
- 6: Träger
- 7: Griffring
- 8: Kugellager
- 9: Kugeln
- 10: Ringschulter
- 11: Spannbackenträger
- 12: Führungen
- 13: Spannbacken
- 14: Sockelabschnitt
- 15: Druckspindel
- 16: Stirnseite
- 17: Durchgangsbohrung
- 18: Durchgangskanal
- 19: Außengewinde
- 20: Hohlraum
- 21: Spülkanal
- 22: Bund
- 23: Stufe
- 24: Spüldorn
- 25, 26: Spülkanal
- 27, 28: Spülfluidaustrittsöffnung
- 29: Spülfluidfontäne
- 30: Marke
- 31: Verzahnung
- 32: Bohrung
- 33: Spülfluidfontäne
- 34: Verschlusselement
- 35: Abschnitt
- 36: Rohrfortsatz
- 37: Ringschulter
- 38: Ringnut
- 39: Faden

## Patentansprüche

1. Spannfutter (1), insbesondere für medizinische Geräte,
mit einem Gehäuse (2), das einen Durchgangskanal (18) aufweist und in dem wenigstens ein Spannelement (13) vorgesehen ist,
mit einer Einrichtung zur Erzeugung einer Spannbewegung des Spannelements (13), wobei die Einrichtung wenigstens ein Getriebeelement (15; 6) aufweist, das mit einem Spülkanal (21; 26) versehen ist, und
mit zumindest drei Spannbacken (13), **dadurch gekennzeichnet, dass** das Getriebeelement von einer Spülkammer (20) umgeben ist, wobei aus der Spülkammer (20) ein Abflusskanal (25, 26) in den Durchgangskanal (18) führt.

2. Spannfutter nach Anspruch 1, **dadurch gekennzeichnet, dass** das Gehäuse (2) mehrteilig aufgebaut ist.

3. Spannfutter nach Anspruch 1, **dadurch gekennzeichnet, dass** die Spannbacken (13) um den Durchgangskanal (18) herum angeordnet sind.

4. Spannfutter nach Anspruch 1, **dadurch gekennzeichnet, dass** das Getriebeelement (15) zur Bewegung der spannbacken (13) vorgesehen ist.

5. Spannfutter nach Anspruch 4, **dadurch gekennzeichnet, dass** das Getriebeelement (15) eine Druckspindel in Form einer Schraubhülse ist.

6. Spannfutter nach Anspruch 5, **dadurch gekennzeichnet, dass** der Spülkanal (21) in der Druckspindel (15) ausgebildet ist und von ihrem zentralen inneren Kanal (17) an ihre Außenseite führt.

7. Spannfutter nach Anspruch 1, **dadurch gekennzeichnet, dass** das Getriebeelement (15) die Spannbacken (13) trägt.

8. Spannfutter nach Anspruch 1, **dadurch gekennzeichnet, dass** in dem Durchgangskanal (18) ein Sitz (23) für einen Spüldorn (24) ausgebildet ist.

9. Spannfutter nach Anspruch 8, **dadurch gekennzeichnet, dass** der Sitz (23) zwischen dem Spülkanal (21) und dem Abflusskanal (26) angeordnet ist.

10. Spannfutter nach Anspruch 1, **dadurch gekennzeichnet, dass** der Spülkanal (21) ein Zuflusskanal ist, der dazu dient, Spülfluid in die Spülkammer (20) des Spannfutters (1) einzuleiten.

11. Spannfutter nach Anspruch 1, **dadurch gekennzeichnet, dass** der Spülkanal (26) ein Abflusskanal ist, der dazu dient, Spülfluid aus der Sprülkammer (20) des Spannfutters (1) auszuleiten.

12. Spannfutter nach Anspruch 1, **dadurch gekennzeichnet, dass** ein Spaunbacken (13) mit einer Markierung zur Einstellung einer Waschposition versehen ist.

13. Verfahren zum Reinigen eines Spannfutters mit einer Spülkammer (20) nach einem der Ansprüche 1 bis 12, **dadurch gekennzeichnet, dass** die spülkammer (20) mittels zumindest eines Spülkanals (21; 26) mit Spülfluid durchströmt wird.

14. Verfahren zum Reinigen eines Spannfutters nach Anspruch 13, **dadurch gekennzeichnet, dass** das Spannfutter (1) von einem medizinischen Gerät gelöst, in eine vorgegebene Einstellposition überführt, auf einen Spüldorn (24) aufgesetzt und durch Zuleitung von Waschfluid durchspült wird.

## Claims

1. Chuck (1), in particular for medical devices,
with a housing (2), which has a through channel (18) and in which at least one clamping element (13) is provided,
with a device for generating a clamping movement of the clamping element (13), wherein the device has at least one gear element (15; 6), which is provided with a rinsing channel (21; 26), and
with at least three clamping jaws (13),
**characterised in that** the gear element is surrounded by a rinsing chamber (20), wherein a discharge channel (25, 26) runs out of the rinsing chamber (20) into the through channel (18).

2. Chuck according to claim 1, **characterised in that** the housing (2) is configured in multiple parts.

3. Chuck according to claim 1, **characterised in that** the clamping jaws (13) are arranged around the through channel (18).

4. Chuck according to claim 1, **characterised in that** the gear element (15) is provided for movement of the clamping jaws (13).

5. Chuck according to claim 4, **characterised in that** the gear element (15) is a pressure spindle in the form of a collet.

6. Chuck according to claim 5, **characterised in that** the rinsing channel (21) is configured in the pressure spindle (15) and runs from its central inner channel (17) onto its outer surface.

7. Chuck according to claim 1, **characterised in that** the gear element (15) carries the clamping jaws (13).

8. Chuck according to claim 1, **characterised in that** a seating (23) for a rinsing mandrel (24) is configured in the through channel (18).

9. Chuck according to claim 8, **characterised in that** the seating (23) is arranged between the rinsing channel (21) and the discharge channel (26).

10. Chuck according to claim 1, **characterised in that** the rinsing channel (21) is an inlet channel, which serves to introduce rinsing fluid into the rinsing chamber (20) of the chuck (1).

11. Chuck according to claim 1, **characterised in that** the rinsing channel (21) is an outlet channel, which serves to direct rinsing fluid out of the rinsing chamber (20) of the chuck (1).

12. Chuck according to claim 1, **characterised in that** a clamping jaw (13) is provided with a marking for adjustment of a wash position.

13. Method for cleaning a chuck with a rinsing chamber (20) according to one of claims 1 to 12, **characterised in that** rinsing fluid is passed through the rinsing chamber (20) by means of at least one rinsing channel (21; 26).

14. Method for cleaning a chuck according to claim 13, **characterised in that** the chuck (1) is released from a medical device, moved into a predetermined adjustment position, placed on a rinsing mandrel (24) and flushed by supplying washing fluid.

## Revendications

1. Mandrin de serrage (1), en particulier pour des appareils médicaux,
comprenant un carter (2) qui présente un canal de passage (18) et dans lequel est prévu au moins un élément de serrage (13),
comprenant un dispositif pour créer un mouvement de serrage de l'élément de serrage (13), le dispositif présentant au moins un élément de transmission (15; 6) qui est doté d'un canal de rinçage (21; 26), et
comprenant au moins trois mors de serrage (13),
**caractérisé en ce que** l'élément de transmission est entouré d'une chambre de rinçage (20), un canal d'évacuation (25, 26) menant de la chambre de rinçage (20) dans le canal de passage (18).

2. Mandrin der serrage selon la revendication 1, **caractérisé en ce que** le carter (2) est constitué de plusieurs parties.

3. Mandrin de serrage selon la revendication 1, **caractérisé en ce que** les mors de serrage (13) sont disposés autour du canal de passage (18).

4. Mandrin de serrage selon la revendication 1, **caractérisé en ce que** l'élément de transmission (15) est prévu pour imprimer un mouvement aux mors de serrage (13).

5. Mandrin de serrage selon la revendication 4, **caractérisé en ce que** l'élément de transmission (15) est une broche de poussée se présentant sous la forme d'une douille filetée.

6. Mandrin de serrage selon la revendication 5, **caractérisé en ce que** le canal de rinçage (21) est réalisé dans la broche de poussée (15) et mène du canal interne central (17) de celle-ci à sa face externe.

7. Mandrin de serrage selon la revendication 1, **caractérisé en ce que** l'élément de transmission (15) porte les mors de serrage (13).

8. Mandrin de serrage selon la revendication 1, **caractérisé en ce qu'**un appui (23) pour un tube de rinçage (24) est réalisé dans le canal de passage (18).

9. Mandrin de serrage selon la revendication 8, **caractérisé en ce que** l'appui (23) est disposé entre le canal de rinçage (21) et le canal d'évacuation (26).

10. Mandrin de serrage selon la revendication 1, **caractérisé en ce que** le canal de rinçage (21) est un canal d'arrivée qui sert à introduire du fluide de rinçage dans la chambre de rinçage (20) du mandrin de serrage (1).

11. Mandrin de serrage selon la revendication 1, **caractérisé en ce que** le canal de rinçage (26) est un canal d'évacuation qui sert à évacuer un fluide de rinçage de la chambre de rinçage (20) du mandrin de serrage (1).

12. Mandrin de serrage selon la revendication 1, **caractérisé en ce qu'**un mors de serrage (13) est pourvu d'un repère pour régler une position de lavage.

13. Procédé de nettoyage d'un mandrin de serrage, comprenant une chambre de rinçage (20), selon une des revendications 1 à 12, **caractérisé en ce que** la chambre de rinçage (20) est traversée par un fluide de rinçage, à l'aide d'au moins un canal de rinçage (21; 26).

14. Procédé de nettoyage d'un mandrin de serrage selon la revendication 13, **caractérisé en ce que** le mandrin de serrage (1) est séparé d'un appareil médical, est transféré dans une position de réglage prédéterminée, est rapporté sur un tube de rinçage (24) et est rincé par introduction d'un fluide de lavage.
